# EUROPÄISCHE PATENTSCHRIFT

(11) **EP 3 006 022 B1**
(45) Veröffentlichungstag und Bekanntmachung des Hinweises auf die Patenterteilung: **13.10.2021**
(21) Anmeldenummer: 15189573.7
(22) Anmeldetag: 25.08.2008
(51) Int. Cl.: A61K 31/185, A61K 31/66, A61K 31/505, A61K 31/205, A61P 11/00, A61P 11/02, A61P 31/12, A61P 31/16

(54) **OSMOLYTE ZUR BEHANDLUNG VON VIRAL BEDINGTEN ATEMWEGSERKRANKUNGEN**
OSMOLYTES FOR THE TREATMENT OF VIRAL RESPIRATORY DISEASES
OSMOLYTE DESTINE AU TRAITEMENT DE MALADIES DES VOIES RESPIRATOIRES D'ORIGINE VIRALE

(30) Priorität: 27.08.2007 DE 102007040615
(43) Veröffentlichungstag der Anmeldung: 13.04.2016
(62) Teilanmeldung aus: 11004754.5
(73) Patentinhaber: bitop AG, 58453 Witten (DE)
(72) Erfinder: Krutmann, Jean, 40237 Düsseldorf (DE); Lentzen, Georg, 46483 Wesel (DE); Schwarz, Thomas, Verstorben (DE)
(74) Vertreter: Schöneborn, Holger

(56) Entgegenhaltungen:
- WO-A-00/76528
- WO-A-2005/002556
- WO-A-2008/031028
- CN-A- 1 813 849
- DE-T2- 69 213 874
- None

## Beschreibung

Osmolyte aus extremophilen Mikroorganismen bilden eine bekannte Gruppe niedermolekularer Schutzstoffe. Extremophile sind sehr außergewöhnliche Mikroorganismen, da sie optimal z.B. bei hohen Salzkonzentrationen (bis 200 g NaCl/l) und hohen Temperaturen (60-110°C) wachsen, die bei mesophilen ("normalen") Organismen zu massiven Schädigungen zellulärer Strukturen führen würden. In den letzten Jahren wurde daher ein großer Forschungsaufwand betrieben, um die biochemischen Komponenten zu identifizieren, die zu der bemerkenswerten thermischen, chemischen und physikalischen Stabilisierung der Zellstrukturen führen. Obwohl viele Enzyme aus hyperthermophilen Mikroorganismen auch unter hohen Temperaturen stabil sind, gilt dies nicht generell für die zellulären Strukturen thermo- und hyperthermophiler Organismen. Zu der hohen Temperaturstabilität von Zellstrukturen tragen in erheblichem Maß niedermolekulare organische Substanzen (Kompatible Solute, Osmolyte) im intrazellulären Milieu bei. Verschiedene neuartige Osmolyte konnten in den letzten Jahren in extremophilen Mikroorganismen erstmals identifiziert werden. In einigen Fällen konnte der Beitrag dieser Verbindungen zum Schutz zellulärer Strukturen gegenüber Hitze und Trockenheit bereits gezeigt werden (Lippert, K., Galinski, E.A. (1994), Appl. Microbiol. Biotech. 37, 61-65; Louis, P., Trüper, H.G., Galinski, E.A. (1994), Appl. Microbiol. Biotech. 41, 684-688; Ramos, Raven, Sharp, Bartolucci, Rossi, Cannio, Lebbink, v. d. Oost, de Vos, Santos (1997), Appl. Environm. Microbiol. 63, 4020-4025; Da Costa, Santos, Galinski (1998), Adv. In Biochemical Engineering Biotechnology, 61, 117-153).

Die in den extremophilen Mikroorganismen gefundenen Osmolyte (kompatible Solute) werden nicht von menschlichen oder tierischen Zellen gebildet.

### Virale Atemwegserkrankungen

Die Rhinoviren sind Erreger, die den sogenannten Schnupfen oder auch Erkältung genannt erzeugen. Sie gehören zur Virusgruppe Picornaviridae (Name von pico = klein und RNA) und bilden dort den Genus *Rhinovirus.* Es werden bis heute 117 Serotypen unterschieden.

Rhinoviren infizieren die Schleimhäute des Nasen- und Rachenraums, bleiben streng lokalisiert und verursachen keine generalisierte Infektion. Es entsteht ein banaler Schnupfen, seltener bei Kindern eine Bronchitis. Der menschliche Körper reagiert auf die Virenattacken mit einer Entzündungsreaktion der Nasenschleimhaut. Die Gefäße der Schleimhaut werden durchlässiger, Flüssigkeit tritt aus, die Nase läuft. Später schwillt die Nasenschleimhaut an auf bis zu einem halben Zentimeter Dicke, wodurch das Atmen durch die Nase so gut wie unmöglich wird. Hinzu kommen eventuell Unwohlsein und Kopfschmerzen. Häufig tritt neben der viruellen Infektion noch eine Sekundärinfektion durch Bakterien im Hals und Rachenraum auf.

Humane Adenoviren sind Viren aus der Familie der Adenoviridae. Viren aus dieser Familie infizieren sowohl Menschen als auch Tiere. Erstmalig wurden sie aus menschlichen Rachenmandeln (Adenoiden) isoliert, davon wurde auch der Name dieser Viren abgeleitet.

Adenoviren verursachen hauptsächlich Erkrankungen der Atemwege. Abhängig vom jeweiligen Serotyp können allerdings auch eine Reihe anderer Erkrankungen hervorgerufen werden, so beispielsweise Gastroenteritis, Konjunktivitis, Zystitis, Rhinitis, Pharyngitis oder Durchfälle. Die Symptome der Atemwegserkrankung durch Adenoviren reichen von der einfachen Erkältung über die Bronchitis bis zur Pneumonie. Bei Patienten mit geschwächtem Immunsystem besteht eine besondere Anfälligkeit für ernsthafte Komplikationen der Adenoviren-Infektionen, wie zum Beispiel das ARDS oder *Acute Respiratory Distress Syndrome.*

Eine überraschenderweise durch Ectoin behandelbare Indikation ist die Anwendung von Osmolyten zur Prophylaxe von Rhinovirus- und/oder Adenovirus-Infektionen des Respirationstraktes. Virusinfektionen, insbesondere Rhinovirusinfektionen, sind eine Hauptursache für die Exazerbation (Verschlimmerung) von Asthma. Man weiß seit einigen Jahren, dass das ICAM-1-Molekül nicht nur als Adhäsionsmolekül für andere Zellen fungiert, sondern zudem als Rezeptor für Rhinoviren (= Schnupfenviren). Zudem wird durch die Rhinovirusinfektion die verstärkte Expression von ICAM-1 in respiratorischen Epithelien ausgelöst. Insofern kann durch die Osmolytbehandlung eine Aufregulation von ICAM-1-Molekülen im Nasenepithel und damit die Expression dieses Rhinovirus-Rezeptors verhindert bzw. abgeschwächt werden, so dass die Entwicklung und Entstehung einer Rhinovirus-Infektion beim Menschen verhindert oder abgeschwächt werden kann. Innerhalb des Adhäsionskomplexes der Zellen befindet sich der CAR-Rezeptor, welcher als Andockstelle der Adenoviren genutzt wird. Die verschiedenen Serotypen der Adenoviridae nutzen dann unterschiedliche weitere Rezeptoren (Integrine, CD46, Heparan-Sulfat-Glykosaminglykane, CD80, CD86 und Mitglieder des MHC-1) um in die Zellen einzudringen. Die Expressionsveränderung von Adhäsionsmolekülen durch Osmolytbehandlung kann also auch eine Möglichkeit der Adenoviren, an die Zelle anzudocken oder einzudringen, abschwächen oder unter Umständen auch verhindern.

Überraschenderweise wurde zudem gefunden, dass die topische Verabreichung mit Hilfe eines Nasensprays auf den Nasenepithelien aus verschiedenen Gründen vorteilhaft ist. Die Verabreichung kann dabei simultan, sequentiell oder separat erfolgen. Die topische Verabreichung des osmolythaltigen Nasensprays sorgt für schnelle Beseitigung der akuten Symptome (z.B. Reizung, Juckreiz, Schwellung), ohne das Nebenwirkungen auftreten. Mit dem in der Zubereitung enthaltenem Osmolyt kann die dem Krankheitsbild zugrunde liegende Entzündung erfolgreich bekämpft werden. Vorteilhaft ist, dass die erfindungsgemäße Kombination auch zur Behandlung von Erkrankungen der oberen und unteren Atemwege eingesetzt werden kann.

Überraschenderweise wurde zusätzlich gefunden, dass die Nebenwirkungen wirkstoffhaltiger Nasensprays (z.B., aber nicht ausschließlich, Glucocorticoide, Antihistaminika) durch die Beimischung von Osmolyten deutlich reduziert werden können. Hierbei könnten die Osmolyte mit anderen Wirkprinzipien kombiniert angewendet werden und reduzieren so die notwendige Einsatzkonzentration des anderen Stoffes. Eine andere Möglichkeit wäre die präventive Gabe von Osmolyten, welche direkt das Nebenwirkunsprofil der später zu gebenden Stoffe schwächen. Dies macht die generelle Kombination von Extremolyten mit Steroiden interessant. Somit ist auch eine Kombinationstherapie von Osmolyten (insbesondere Ectoin und Hydroxyectoin) mit anderen intranasal oder intraocular verabreichten Wirkstoffen, die unerwünschte Nebenwirkungen verursachen, sinnvoll. Hierbei ist auch vorstellbar, Osmolyten Wirkstoffformulierungen beizumischen, die nasal oder ocular zur Therapie einer Organkrankheit (z.B. Krebs) verabreicht werden, aber eine entzündliche Nebenwirkung auf das Nasenepithel entfalten. Die gemeinsame Gabe von Osmolyten mit z.B. Glucocorticoiden oder Antihistaminika bzw. die präventive Gabe von Osmolyten vor einer Therapie mit diesen Stoffen könnte als allgemeines Prinzip auch in anderen Applikationsformen (topisch, dermal, intraperitoneal, intravenös, intramuskulär, oral) eine effektive Möglichkeit sein, die Nebenwirkungen der Stoffe zu senken oder durch die Kombination der Wirkprinzipien von Osmolyten mit denen der gleichzeitig oder danach applizierten Wirkstoffe die Einsatzkonzentration der Wirkstoffe zu senken, wodurch eine geringe Belastung des behandelten Patienten entsteht.

Bei den kompatiblen Soluten (Osmolyten) handelt es sich um Ectoin, Hydroxyectoin und/oder Salze, Ester oder Säuren dieser Verbindungen. Die Konzentration der kompatiblen Solute liegt typischerweise zwischen 0,01 und 20 Gew.-%, bevorzugt zwischen 0,1 und 10 Gew.-%, besonders bevorzugt zwischen 0,1 und 5 Gew.-%, bezogen auf das Gesamtgewicht.

Durch eine auf die jeweilige Behandlung, Applikation und Indikation abgestimmte Verabreichung der Osmolyte können die wirksamen Konzentrationen bereits bei niedrigen Dosierungen erreicht werden. Die Gabe des Osmolyts ermöglicht die Bekämpfung der lästigen Reaktionen wie Juckreiz, Nasenfluss und verhindert das Fortschreiten der Entzündung. Somit ist eine bessere Compliance der Patienten zu erwarten.

Insbesondere bei der intranasalen oder intraocularen Verabreichung kommt es nicht nur zu einer schnellen Wirkung, sondern auch zu einer hohen therapeutischen Wirksamkeit, die mit einer starken antientzündlichen Wirkung einhergeht. Der vorliegenden Erfindung liegt daher die Aufgabe zugrunde, Mittel zur Prävention oder Therapie von immunologischen Überempfindlichkeitsreaktionen, insbesondere zur Prävention von Rhinovirus- und/oder Adenovirus-Infektionen zur Verfügung zu stellen, die einen breiteren (bewusst nicht monospezifischen), nebenwirkungsarmen und damit einen effektiveren Therapieansatz darstellen. Ein Aspekt der vorliegenden Erfindung besteht in der Verwendung von Osmolyten zur Herstellung eines Arzneimittels oder Medizinproduktes z.B. in Form eines Nasensprays oder in Form von Augentropfen zur Prävention oder Therapie von Virusinfektionen, indem es durch den osmolytvermittelten Schutz der Nasenepithelzellen zu einer Abschwächung der Expression pro-inflammatorischer Genprodukte (z.B. ICAM1) im Rahmen von Entzündungsreaktionen kommt.

Die Dosierung kann mehrmals täglich erfolgen, wobei die Einzeldosis von dem verwendeten Osmolyten und insbesondere vom allgemeinen Zustand des Patienten (Alter, Gewicht, etc.) und dem Schweregrad der Erkrankung abhängt.

Für die topische Anwendung können verschiedene pharmazeutische Formulierungen, z.B. Nasensprays, Nasentropfen und Augentropfen, in Frage kommen. Aufgrund der Wasserlöslichkeit des Wirkstoffes Osmolyt (bevorzugt Ectoin) können Formulierungen mit diesen Wirkstoffen vorzugsweise als wäßrige Lösungen formuliert werden.

Neben den wirksamen Osmolyten können die erfindungsgemäßen pharmazeutischen Zubereitungen weitere Bestandteile wie Konservierungsstoffe, Stabilisatoren, Isotonisierungsmittel, Verdickungsmittel, Suspensionsstabilisatoren, Hilfstoffe zur pH-Einstellung, Puffersysteme und Netzmittel enthalten. Die erfindungsgemäßen pharmazeutischen Zubereitungen können zudem weitere wirksame Bestandteile wie Antihistaminika oder Steroidwirkstoffe (z. B. Loteprednoletabonat) enthalten.

Als Konservierungsmittel kommen in Frage: Benzalkoniumchlorid, Chlorbutanol, Thiomersal, Methylparaben, Propylparaben, Sorbinsäure und deren Salze, Natriumedetat, Phenylethyl-alkohol, Chlohexidinhydrochloridacetat, -digluconat, Cetylpyridiniumchlorid, -bromid, Chlorkresol, Phenylquecksilberacetat, Phenylquecksilbernitrat, Phenyquecksilberborat, Phenoxyethanol.

Geeignete Hilfsstoffe zur Einstellung der Isotonie der Formulierungen sind beispielsweise: Natriumchlorid, Kaliumchlorid, Mannitol, Glucose, Sorbitol, Glycerol, Propylenglycol. Im Allgemeinen werden diese Hilfsstoffe in Konzentrationen von 0,1 bis 10% eingesetzt.

Die Formulierungen der Erfindung können ebenfalls geeignete Puffersysteme oder andere Hilfsstoffe zur pH-Einstellung beeinhalten, um einen pH-Wert einzustellen und aufrechtzuerhalten in der Größenordnung von 4-8, vorzugsweise von 5 bis 7,5. Geeignete Puffersysteme sind Citrat, Phosphat, Tromethamol, Glycin, Borat, Acetat. Diese Puffersysteme können hergestellt werden aus Substanzen wie Citronensäure, Mononatriumphosphat, Dinatriumphosphat, Glycin, Borsäure, Natriumtetraborat, Essigsäure oder Natriumactat.

Es können ebenfalls weitere Hilfsstoffe zur pH-Einstellung verwendet werden wie Salzsäure oder Natriumhydroxid. Als Netzmittel für die Formulierungen kommen in Frage: Benzalkoniumchlorid, Cetylpyridiniumchlorid, Tyloxapol, verschiedene Polysorbate [Tween⁽™⁾] sowie weitere polyoxyethylierte Substanzen und Poloxamere.

Das nachfolgende Beispiel eines Nasensprays illustriert die Erfindung, ohne diese einzuschränken.

### Nasenspray mit Ectoin (0,1 %)

In einem geeigneten Rührwerksbehälter ca. 45 kg gereinigtes Wasser vorlegen. Darin den Wirkstoff Ectoin, Hydroxypropylmethylcellulose, Natriumacetat, Benzalkoniumchlorid und Sorbitollösung nacheinander zugeben und unter Rühren auflösen. Die entstandene Lösung mit gereinigtem Wasser auf ein Volumen von 49,5 Liter auffüllen. Den pH-Wert der Lösung mit 1 N Natronlauge auf pH 6,0 einstellen. Mit gereinigtem Wasser auf das Endvolumen von 50,0 Liter auffüllen und rühren. Die Lösung durch einen geeigneten Filter filtrieren und in Flaschen abfüllen, welche anschließend mit einer geeigneten Nasenspraypumpe versehen werden.

### Wirksamkeitsstudien

### Hemmung der ICAM-1 Expression durch Ectoin

Die Expression von ICAM-1 wurde mit der differential reverse transcriptase-PCR (RT-PCR) und dem Kit von Applied Biosystem gemessen. Um die normalen Schwankungen in der Genexpression der Hautzellen zu berücksichtigen, wird die ICAM-1 Expression im Verhältnis zu dem konstitutiv gebildeten house-keeping Gen ß-Aktin ins Verhältnis gesetzt. Die semiquantitative Analyse der RT-PCR wurde mit einer lonenaustauscherchromatographie mit einem UV-Spektrophotometer (A260) durchgeführt. (A) Nicht vorbehandelte, bestrahlte Kontrolle, (B) mit 1 mM RonaCareTMEctoin 24 h vorinkubiert und mit einer Einzeldosis von 30 J/cm² bestrahlt oder (C) mit 1 mM Ectoin 24 h vorinkubiert, unbestrahlt dem Zellmedium 24 h vorinkubiert. Die UVA-Strahlung induziert eine Aufregulation der ICAM-1-Expression. Die Vorbehandlung von Keratinozyten mit 1 mM Ectoin kann die durch UVA-Strahlung induzierte ICAM-1-Induktion zu allen Zeitpunkten fast vollkommen aufheben.

## Patentansprüche

1. Medikament enthaltend als Wirkstoff mindestens ein kompatibles Solut zur Anwendung in der Prävention und/oder Behandlung von viral bedingten Atemwegserkrankungen, wobei das kompatible Solut ausgewählt ist aus der Gruppe bestehend aus: Ectoin, Hydroxyectoin und/oder Salzen oder Estern dieser Verbindungen.

2. Medikament zur Anwendung nach Anspruch 1, **dadurch gekennzeichnet, dass** die Atemwegserkrankung eine Erkältung, Rhinitis, Schnupfen, Bronchitis, Grippe, Pneumonie oder durch Adenoviren verursachte Pharyngitis ist.

3. Medikament zur Anwendung nach Anspruch 1 oder 2, **dadurch gekennzeichnet, dass** das Medikament der Prophylaxe von Rhinovirus- und/oder Adenovirus-Infektionen des Respirationstrakts dient.

4. Medikament zur Anwendung nach einem der Ansprüche 1 bis 3, **dadurch gekennzeichnet, dass** das kompatible Solut in einer Konzentration von 0,01 bis 20 Gew.-%, bevorzugt 0,1 bis 10 Gew.-%, besonders bevorzugt 0,1 bis 5 Gew.-% bezogen auf das Gesamtgewicht vorliegt.

5. Medikament zur Anwendung nach einem der Ansprüche 1 bis 4, **dadurch gekennzeichnet, dass** das Medikament in flüssiger oder halbflüssiger Applikationsform vorliegt.

6. Medikament zur Anwendung nach einem der Ansprüche 1 bis 5, **dadurch gekennzeichnet, dass** das Medikament in Form eines Sprays, in Form von Augentropfen oder Nasentropfen oder als inhalierbare flüssige oder feste Zubereitung vorliegt.

## Claims

1. Medicament containing as active substance at least one compatible solute for use in the prevention and/or treatment of viral respiratory diseases, the compatible solute being selected from the group consisting of: ectoine, hydroxyectoine and/or salts or esters of these compounds.

2. Medicament for the use according to claim 1, **characterised in that** the respiratory disease is a common cold, rhinitis, coryza, bronchitis, influenza, pneumonia or adenovirus-induced pharyngitis.

3. Medicament for the use according to claim 1 or 2, **characterised in that** the medicament is used for the prophylaxis of rhinovirus and/or adenovirus infections of the respiratory tract.

4. Medicament for the use according to one of claims 1 to 3, **characterised in that** the compatible solute is present in a concentration of 0.01 to 20 wt.%, preferably 0.1 to 10 wt.%, particularly preferably 0.1 to 5 wt.%, relative to the total weight.

5. Medicament for the use according to one of claims 1 to 4, **characterised in that** the medicament is present in a liquid or semi-liquid administration form.

6. Medicament for the use according to one of claims 1 to 5, **characterised in that** the medicament is present in the form of a spray, in the form of eye drops or nose drops, or as an inhalable liquid or solid preparation.

## Revendications

1. Médicament contenant en tant que principe actif au moins un soluté compatible destiné à être utilisé dans la prophylaxie et/ou le traitement de maladies respiratoires virales, dans lequel le soluté compatible est choisi parmi le groupe consistant en ectoïne, hydroxyectoïne et/ou sels ou esters desdits composés.

2. Médicament destiné à être utilisé selon la revendication 1, **caractérisé en ce que** la maladie respiratoire est un rhume, une rhinite, une bronchite, une grippe, une pneumonie ou une pharyngite provoquée par des adénovirus.

3. Médicament destiné à être utilisé selon la revendication 1 ou 2, **caractérisé en ce que** le médicament sert à la prophylaxie d'infections dues à un rhinovirus et/ou à un adénovirus des voies respiratoires.

4. Médicament destiné à être utilisé selon l'une quelconque des revendications 1 à 3, **caractérisé en ce que** le soluté compatible est présent en une concentration de 0,01 à 20 % en poids, de manière préférée de 0,1 à 10 % en poids, de manière particulièrement préférée de 0,1 à 5 % en poids par rapport au poids total.

5. Médicament destiné à être utilisé selon l'une quelconque des revendications 1 à 4, **caractérisé en ce que** le médicament est présent sous une forme d'application liquide ou semi-liquide.

6. Médicament destiné à être utilisé selon l'une quelconque des revendications 1 à 5, **caractérisé en ce que** le médicament est présent sous la forme d'un spray, sous la forme d'un collyre ou de gouttes nasales ou en tant qu'une préparation liquide ou solide inhalable.
